(19) Europäisches Patentamt / European Patent Office / Office européen des brevets

(11) **EP 3 590 428 B1**

(12) **EUROPÄISCHE PATENTSCHRIFT**

(45) Veröffentlichungstag und Bekanntmachung des Hinweises auf die Patenterteilung:
**19.10.2022 Patentblatt 2022/42**

(21) Anmeldenummer: **18182181.0**

(22) Anmeldetag: **06.07.2018**

(51) Internationale Patentklassifikation (IPC):
**A61B 6/00** *(2006.01)* **G06T 5/50** *(2006.01)*

(52) Gemeinsame Patentklassifikation (CPC):
**A61B 6/5235; A61B 6/481; A61B 6/487; A61B 6/504; G06T 5/50;** A61B 6/4441; A61B 6/463; A61B 6/5258; A61B 6/54

(54) **VERFAHREN UND BILDGEBENDES GERÄT ZUM ERZEUGEN EINES ROADMAP-BILDES**

METHOD AND IMAGING APPARATUS FOR GENERATING A ROAD MAP IMAGE

PROCÉDÉ ET APPAREIL D'IMAGERIE PERMETTANT DE GÉNÉRER UNE IMAGE DE FEUILLE DE ROUTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**08.01.2020 Patentblatt 2020/02**

(73) Patentinhaber: **Siemens Healthcare GmbH 91052 Erlangen (DE)**

(72) Erfinder:
• **Böhm, Stefan**
  **90522 Oberasbach (DE)**
• **Bernhardt, Philipp**
  **91301 Forchheim (DE)**
• **Berting, Andreas**
  **36381 Schlüchtern (DE)**

(56) Entgegenhaltungen:
EP-A1- 3 320 843    US-A1- 2009 180 591
US-A1- 2009 180 676    US-A1- 2017 228 863

**Beschreibung**

Die Erfindung betrifft ein Verfahren zum Erzeugen eines Roadmap-Bildes eines Zielobjekts mittels eines bildgebenden Geräts und ein entsprechend eingerichtetes bildgebendes Gerät. Das bildgebende Gerät kann insbesondere ein medizinisches bildgebendes Gerät sein, wie etwa ein Röntgengerät. Ein Roadmap-Bild im Sinne der vorliegenden Erfindung ist ein nach dem an sich insbesondere in dem technischen Feld medizinischer Bildgebung bekannten Roadmap-Verfahren erzeugtes Bild.

Bei dem Roadmap-Verfahren werden üblicherweise zwei Bilder einander überlagert, um eine Orientierung oder Navigation zu erleichtern. Diese zwei Bilder sind beispielsweise ein Live-Röntgenbild oder Fluoroskopie-Bild, welches eine jeweils aktuelle Situation oder einen jeweils aktuellen Zustand des Zielobjekts zeigt und ein Orientierungsbild. Weiter handelt es sich bei dem Roadmap-Verfahren im Sinne der vorliegenden Erfindung um ein Doppelsubtraktionsverfahren. Das bedeutet, dass die beiden zum Erzeugen des Roadmap-Bildes einander überlagerten Bilder selbst jeweils Subtraktionsbilder oder subtrahierte Bilder sind. Insbesondere werden dabei 2D-Bilder verwendet.

Zum Beispiel beschreibt das Dokument US2017/0228863 A1 ein Navigationsverfahren bei Eingriffen in den menschlichen Körper unter Verwendung einer Roadmaptechnik. Dabei wird ein erstes Subtraktionsbild erzeugt, dass ausschließlich Blutgefäße des Patienten zeigt und ein zweites Subtraktionsbild, das ausschließlich ein medizinisches Instrument zeigt. Die Subtraktionsbilder werden überlagert, sodass in dem entstehenden Überlagerungsbild das medizinische Instrument in dem Blutgefäßsystem sichtbar ist.

Allgemein besteht bei der Anwendung des Roadmap-Verfahrens die Problematik, dass bei Anwendung beispielsweise einer Zoomfunktion sich jeweilige physikalische Bedingungen einer Bildaufnahme verändern, sodass die Subtraktionsbilder nicht artefaktfrei erzeugt oder überlagert werden können. Damit sind derartige Funktionen des bildgebenden Geräts bisher während des Roadmap-Verfahrens praktisch nicht oder zumindest nicht mit einem Informationsgewinn anwendbar. Heutzutage wird daher beispielsweise lediglich ein Digitalzoom eingesetzt, durch welchen zwar keine Bildaufnahmebedingungen verändert werden und damit auch keine Subtraktions- oder Überlagerungsartefakte entstehen, welcher andererseits aber auch keinen objektiven Informationsgewinn, beispielsweise keine verbesserte Detailauflösung oder dergleichen mit sich bringt.

Aufgabe der vorliegenden Erfindung ist es, eine flexiblere Anwendbarkeit des Roadmap-Verfahrens mit verbesserter Bildqualität zu ermöglichen. Diese Aufgabe wird erfindungsgemäß durch die Gegenstände der unabhängigen Patentansprüche gelöst. Vorteilhafte Weiterbildungen und Ausgestaltungen der Erfindung sind in den abhängigen Patentansprüchen, in der Beschreibung und in den Zeichnungen angegeben.

Ein erfindungsgemäßes Verfahren dient zum Erzeugen wenigstens eines neuen Roadmap-Bildes eines mittels eines bildgebenden Geräts, insbesondere mittels eines medizinischen bildgebenden Geräts, abgebildeten Zielobjekts. Das bildgebende Gerät kann bevorzugt ein Röntgengerät, insbesondere ein Fluoroskopiegerät, sein. Das Zielobjekt kann letztlich ein beliebiges zur Abbildung oder Darstellung durch das bildgebende Gerät geeignetes Objekt sein. Das erfindungsgemäße Verfahren kann also beispielsweise sowohl in einem medizinischen Umfeld zum Abbilden eines Patienten oder eines biologischen Gewebes als auch beispielsweise in einem industriellen Umfeld angewendet werden.

Beispielsweise ein Injizieren oder Einbringen eines Kontrastmittels oder dergleichen ist dabei ebenso wie ein gegebenenfalls gleichzeitig mit dem erfindungsgemäßen Verfahren ausgeführter chirurgischer Eingriff explizit nicht Teil des beanspruchten Verfahrens. Das erfindungsgemäße Verfahren ist also explizit kein chirurgisches Verfahren. Vielmehr kann das erfindungsgemäße Verfahren etwa als Verfahren zum Betreiben des bildgebenden Geräts aufgefasst werden. Ebenso kann das erfindungsgemäße Verfahren beispielsweise zum Verarbeiten bereits aufgenommener und bereitgestellter Bilder oder Bilddaten angewendet werden und ist damit unabhängig von einem direkten medizinischen oder chirurgischen Verfahren.

Bei dem erfindungsgemäßen Verfahren wird zunächst ein erstes Roadmap-Bild erzeugt durch Überlagerung eines ersten und eines zweiten Subtraktionsbildes, wobei das erste Subtraktionsbild erzeugt wird aus einem mittels des bildgebenden Geräts aufgenommenen ersten Maskenbild und einem ebenfalls mittels des bildgebenden Geräts aufgenommenen Kontrastmittelbild jeweils des Zielobjekts. Dazu kann das erste Maskenbild beispielsweise vor und das Kontrastmittelbild nach einer Flutung des Zielobjekts mit einem Röntgen-Kontrastmittel aufgenommen werden. Das zweite Subtraktionsbild wird erzeugt aus einem mittels des bildgebenden Geräts aufgenommenen zweiten Maskenbild und einem ebenfalls mittels des bildgebenden Geräts aufgenommenen ersten Untersuchungsbild des Zielobjekts. Als das zweite Maskenbild kann dabei das erste Maskenbild verwendet werden oder es kann separat aufgenommen werden. Das Untersuchungsbild ist ein nach dem Kontrastmittelbild und nach dem zweiten Maskenbild aufgenommenes Bild des Zielobjekts.

Insbesondere werden die vier zum Erzeugen des ersten und zweiten Subtraktionsbildes verwendeten Bilder in der Reihenfolge, in der sie hier genannt sind, aufgenommen. Mit anderen Worten wird also zu einem ersten Zeitpunkt das erste Maskenbild, zu einem späteren zweiten Zeitpunkt das Kontrastmittelbild, zu einem noch späteren dritten Zeitpunkt das zweite Maskenbild und zu einem nochmals späteren vierten Zeitpunkt das erste Untersuchungsbild aufgenommen. Dies gilt insoweit als das das erste Maskenbild nicht als das zweite Maskenbild verwendet wird. Zum Erzeugen der Subtraktionsbilder wird das jeweilige Maskenbild von dem jeweiligen anderen beteiligten Bild abgezogen. Das bedeutet, dass entsprechende Pixel- oder Intensitätswerte der jeweiligen Bilder voneinander subtrahiert werden, sodass die resultierenden Subtraktionsbilder eine Differenz der jeweiligen Bilder zeigen oder darstellen.

Dass die beiden Subtraktionsbilder zum Erzeugen des ersten Roadmap-Bildes einander überlagert werden, kann - muss aber nicht ausschließlich - bedeuten, dass die Subtraktionsbilder beziehungsweise deren Pixel- oder Intensitätswerte ihrer jeweils einander hinsichtlich ihrer Lage, also hinsichtlich eines durch sie abgebildeten Teilbereiches des Zielobjekts, einander entsprechenden Pixel, addiert werden. Ebenso kann die Überlagerung beziehungsweise das Überlagern eine Anwendung einer komplexeren Kombinationsfunktion bedeuten, durch welche eines oder beide der einander überlagerten, also miteinander kombinierten, Subtraktionsbilder zum Erzeugen des Roadmap-Bildes modifiziert werden.

Erfindungsgemäß ist es vorgesehen, dass nach dem Erzeugen des ersten Roadmap-Bildes, beziehungsweise nach dem Aufnehmen der wenigstens vier zum Erzeugen des ersten und zweiten Subtraktionsbildes verwendeten Bilder, ein Parameterwert eines physikalischen Parameters des bildgebenden Geräts verändert wird, welcher das Aufnehmen von Bildern des Zielobjekts mittels des bildgebenden Geräts beeinflusst. Der Parameter, dessen Wert hier verändert wird, ist also ein Bildgebungs- oder Bildaufnahmeparameter des bildgebenden Geräts. Als derartiger Parameter beziehungsweise Parameterwert kann hier beispielsweise eine für die Bildaufnahme verwendete Zoomstufe, eine verwendete Blende oder Blendengröße oder Blendenform, eine Kollimatoreinstellung, eine elektrische Spannung einer Röntgenröhre des bildgebenden Geräts, eine Strahlungsleistung oder Strahlendosis pro Bild und/oder dergleichen mehr verändert werden. Mit anderen Worten führt also die hier vorgesehene Veränderung des Parameterwertes dazu, dass beim Erzeugen eines Subtraktionsbildes aus jeweils einem vor und nach dem Verändern des Parameterwertes aufgenommenen Bild typischerweise Bildartefakte entstehen würden. Insbesondere ist es daher vorgesehen, dass zumindest das erste Maskenbild und das Kontrastmittelbild einerseits sowie das zweite Maskenbild und das erste Untersuchungsbild andererseits mit jeweils gleicher Einstellung des entsprechenden Parameters, also mit gleichen Parameterwerten, das heißt unter gleichen physikalischen Bedingungen hinsichtlich der Bildgebung oder Bildaufnahme, aufgenommen werden. Nach dem Verändern des Parameterwertes aufgenommene Bilder des Zielobjekts werden demgegenüber mit dem neuen, also veränderten Parameterwert und dementsprechend unter veränderten physikalischen Aufnahmebedingungen aufgenommen.

Das Verändern des Parameterwertes kann beispielsweise manuell durch einen Benutzer oder ein Bedienpersonal erfolgen. Ebenso kann das Verändern des Parameterwertes aber beispielsweise automatisch gemäß einem vorgegebenen Programm durch das bildgebende Gerät selbst durchgeführt werden. Allgemein kann das Verändern des Parameterwertes durch das bildgebende Gerät zumindest teilautomatisch auf einen Empfang eines entsprechenden Steuer- oder Datensignals hin durchgeführt werden. Das Verändern des Parameterwertes ist hier beispielhaft zu verstehen, da ebenso mehrere entsprechende Parameterwerte des oder mehrere Parameter verändert werden können.

Unmittelbar nach dem Verändern des Parameterwertes wird erfindungsgemäß unter Verwendung des veränderten Parameterwertes durch das bildgebende Gerät automatisch ein drittes Maskenbild des Zielobjekts aufgenommen. Das dritte Maskenbild bildet also den Zustand des Zielobjekts nach dem Verändern des Parameterwertes ab. Da das dritte Maskenbild mit dem veränderten Parameterwert aufgenommen wurde, kann es vorteilhaft als Maskenbild zur Erzeugung weiterer Subtraktionsbilder aus anschließend ebenfalls mit dem veränderten Parameterwert aufgenommenen weiteren Bildern des Zielobjekts verwendet werden, ohne dass dabei Artefakte, Bildfehler oder dergleichen entstehen. Das automatische Aufnehmen dieses dritten Maskenbildes ist dabei besonders vorteilhaft, da sich somit ein Anwender ganz auf die Untersuchung des Zielobjekts konzentrieren kann, ohne darüber nachdenken zu müssen, ob die vorgenommene Veränderung des Parameterwertes gegebenenfalls zu Bildartefakten führen kann. Dass das dritte Maskenbild unmittelbar nach dem Verändern des Parameterwertes aufgenommen wird, soll im Sinne der vorliegenden Erfindung insbesondere bedeuten, dass das dritte Maskenbild in einem vorgegebenen Zeitraum nach dem Verändern des Parameterwerts, bevorzugt als erstes Bild des Zielobjekts nach dem Verändern des Parameterwerts, aufgenommen wird. Insbesondere wird das dritte Maskenbild beispielsweise aufgenommen bevor weiteres Kontrastmittel, ein Hilfs- oder Fremdobjekt, ein medizinisches Gerät oder dergleichen in einen Aufnahmebereich des bildgebenden Geräts oder in das Zielobjekt selbst eingebracht wird.

Insbesondere kann vorgesehen sein, dass automatisch ein Regelstopp für das bildgebende Gerät eingestellt wird, jeweils nachdem ein Maskenbild aufgenommen wurde. Der Regelstopp kann beispielsweise für eine vorbestimmte Zeitdauer eingestellt, also aufrechterhalten werden und/oder jeweils solange, bis wenigstens ein weiteres Bild, beispielsweise ein jeweiliges Untersuchungsbild, aufgenommen wurde. Der Regelstopp verhindert, dass Änderungen an die Bildgebung oder Bilderfassung, also die Aufnahmebedingungen beeinflussenden Parametern oder Parameterwerten des bildgebenden Geräts vorgenommen werden. Dadurch kann erreicht werden, dass jeweilige Bilder möglichst artefaktfrei überlagert oder kombiniert werden können und beispielsweise für eine bestimmte Parametereinstellung nicht nur ein einziges Bild aufgenommen wird, zu welchem dann andernfalls beispielsweise kein mit der gleichen Parametereinstellung aufgenommenes Maskenbild existiert.

Weiter ist es erfindungsgemäß als Teil des erfindungsgemäßen Verfahrens vorgesehen, dass aus wenigstens einem nach dem dritten Maskenbild mit dem veränderten Parameterwert aufgenommenen zweiten Untersuchungsbild des Zielobjekts durch Subtraktion des dritten Maskenbilds von diesem zweiten Untersuchungsbild wenigstens ein drittes Subtraktionsbild des Zielobjekts erzeugt wird. Dieses Erzeugen des dritten Subtraktionsbildes erfolgt also analog zu dem Erzeugen des zweiten Subtraktionsbildes, wobei jedoch sowohl für das zweite Untersuchungsbild als auch für das dritte Maskenbild der gegenüber dem ersten und zweiten Maskenbild veränderte Parameterwert verwendet wird. Dass wenigstens ein zweites Untersuchungsbild aufgenommen wird und entsprechend wenigstens ein drittes Subtraktionsbild erzeugt wird, bedeutet, dass beispielsweise von jedem nach dem dritten Maskenbild aufgenommenen Untersuchungsbild jeweils das dritte Maskenbild zum Erzeugen eines entsprechenden jeweiligen dritten Subtraktionsbildes abgezogen werden kann.

Dabei können also nach dem dritten Maskenbild mehrere Untersuchungsbilder aufgenommen werden, welche als zweite Untersuchungsbilder oder entsprechend fortlaufend nummeriert bezeichnet werden können, also etwa als zweites Untersuchungsbild, drittes Untersuchungsbild, viertes Untersuchungsbild usw. Dementsprechend kann sich eine Bezeichnung von aus diesen Untersuchungsbildern erzeugten Subtraktionsbildern an der für die Untersuchungsbilder verwendeten Nummerierung oder Nomenklatur orientieren, sodass also mehrere dritte Subtraktionsbilder für unterschiedliche Untersuchungsbilder erzeugt werden können, wobei die mehreren dritten Subtraktionsbilder dann ebenso als beispielsweise drittes Subtraktionsbild, viertes Subtraktionsbild, fünfte Subtraktionsbild usw. bezeichnet werden können.

Das Verfahren wird insbesondere so lange, also für solche Untersuchungsbilder angewendet, für deren Aufnahme derselbe Parameterwert des zuvor veränderten Parameters verwendet wird wie für die Aufnahme des dritten Maskenbildes. Dadurch ist sichergestellt, dass das wenigstens eine dritte Subtraktionsbild beziehungsweise die mehreren dritten Subtraktionsbilder keine aus einer weiteren Veränderung des Parameterwerts, also der physikalischen Aufnahmebedingungen, resultierenden Bildartefakte enthalten.

Weiter ist es erfindungsgemäß vorgesehen, dass das wenigstens eine neue Roadmap-Bild des Zielobjekts nach Veränderung des Parameterwertes erzeugt wird unter Verwendung des ersten Subtraktionsbildes und des wenigstens einen dritten Subtraktionsbildes. Wurden also zuvor mehrere dritte Subtraktionsbilder erzeugt, so kann aus jedem dieser dritten Subtraktionsbilder jeweils unter Verwendung des ersten Subtraktionsbild ein jeweiliges neues Roadmap-Bild erzeugt werden.

Wird nach dem Aufnehmen der zweiten Untersuchungsbildes beziehungsweise nach Erzeugen des dritten Subtraktionsbildes oder des neuen Roadmap-Bildes der Parameterwert wieder auf seinen ursprünglichen oder vorherigen Wert zurückgesetzt, so können aus weiteren danach aufgenommenen Untersuchungsbilder jeweilige Subtraktionsbilder durch Subtraktion des zweiten Maskenbildes oder des ersten Maskenbildes erzeugt und für weitere neue Roadmap-Bilder verwendet werden.

Ebenso kann das erfindungsgemäße Verfahren nach dem beschriebenen Schema iterativ fortgesetzt werden. Das bedeutet, dass nach dem beschriebenen Verändern des Parameterwertes und dem Aufnehmen des zweiten Untersuchungsbildes der Parameterwert beispielsweise erneut verändert werden kann auf einen zuvor nicht verwendeten Wert. In einem solchen Fall können zwischen der ersten und der zweiten Veränderung des Parameterwertes aufgenommene Bilder die entsprechenden Rollen der im vorherigen Iterationsschritt vor der ersten Veränderung des Parameterwerts aufgenommenen Bilder einnehmen. Zumindest kann beispielsweise das dritte Subtraktionsbild dann als zweites Subtraktionsbild gemäß dem zuvor beschriebenen erfindungsgemäßen Verfahren verwendet werden. Das ursprüngliche erste Subtraktionsbild kann gegebenenfalls jedoch als solches auch nach der weiteren oder zweiten Veränderung des Parameterwertes weiterverwendet werden.

Obwohl dies hier nicht im Einzelnen explizit beschrieben ist, können die jeweiligen Bilder, also etwa die Maskenbilder, das Kontrastmittelbild, die Untersuchungsbilder, die Subtraktionsbilder und/oder die Roadmap-Bilder, mittels eines Bildverarbeitungsverfahrens vor- und oder nachbearbeitet sein oder werden. Dadurch kann beispielsweise eine Bildqualität oder Erkennbarkeit einzelner Details verbessert, ein Rauschen unterdrückt und/oder eine Weiterverarbeitung erleichtert werden. Beispielsweise kann vor dem jeweiligen Erzeugen der Subtraktionsbilder auf die jeweils dazu verwendeten Bilder ein logarithmischer Filter angewendet, die Bilder also logarithmiert werden.

Das erfindungsgemäße Verfahren zum Erzeugen eines neuen Roadmap-Bildes ist ein Verfahren oder kann aufgefasst oder eingesetzt werden als Verfahren zum automatischen oder teilautomatischen Betreiben oder Steuern des bildgebenden Geräts, wobei dieses eine entsprechende Bilderzeugungs- oder Datenverarbeitungseinrichtung zum Verarbeiten und Erzeugen der verschiedenen beschriebenen Bilder des Zielobjekts umfasst. Das vorliegend beanspruchte Verfahren ist also explizit kein chirurgisches Verfahren und beansprucht auch keinen chirurgischen Verfahrensschritt, obwohl das erfindungsgemäße Verfahren bevorzugt in einem medizinischen Anwendungsfeld eingesetzt werden kann.

Durch das erfindungsgemäße Verfahren wird vorteilhaft sichergestellt, dass für die genannten Subtraktionsbilder jeweils zwei Bilder verwendet werden, welche mit demselben Parameterwert, also unter Verwendung jeweils desselben Parameterwertes oder derselben Parameterwerte aufgenommen wurden. Dadurch ist also sichergestellt, dass in den beschriebenen Subtraktionsbildern keine entsprechenden Bildfehler oder Artefakte auftreten und somit auch die genannten Roadmap-Bilder eine entsprechend gute oder uneingeschränkte Bildqualität aufweisen. Somit ermöglicht es das erfindungsgemäße Verfahren, veränderte Aufnahmebedingungen oder entsprechende Funktionen, welche eine Veränderung des Parameterwertes erfordern, auch während, also bei Anwendung des Roadmap-Verfahrens zu nutzen. Dabei können wie beschrieben selbstverständlich ebenso mehrere Parameterwerte des physikalischen Parameters oder mehrerer physikalischer Parameter des bildgebenden Geräts in entsprechender Weise verändert werden. Durch das erfindungsgemäße Verfahren kann sich somit vorteilhaft ein Informationsgewinn in einem oder für den nach dem Verändern des Parameterwertes abgebildeten Bereich des Zielobjekts ergeben, beispielsweise in Form einer höheren Auflösung eines Teilbereiches des Zielobjekts und/oder in Form eines größeren Signal-Rausch-Verhältnisses oder dergleichen. Dabei muss insbesondere keine Verschlechterung der Bildqualität in Kauf genommen werden. Gegenüber einem herkömmlich verwendeten Digitalzoom kann somit beispielsweise ein echtes oder optisches Zoom verwendet werden, welcher zu diesem Informationsgewinn führen kann. Somit wird durch das erfindungsgemäße Verfahren letztlich eine genauere, detailliertere, zuverlässigere und/oder sicherere Abbildung, Untersuchung oder Bewertung des Zielobjekts bei Verwendung des Roadmap-Verfahrens ermöglicht.

In vorteilhafter Ausgestaltung der vorliegenden Erfindung wird automatisch ein Hilfssubtraktionsbild erzeugt, indem von einem, insbesondere zuletzt oder unmittelbar, vor dem Verändern des Parameterwertes aufgenommenen Untersuchungsbild des Zielobjekts das zweite Maskenbildes oder das erste Maskenbild subtrahiert wird. Es wird dann automatisch ein kombiniertes Subtraktionsbild erzeugt durch Kombinieren des Hilfssubtraktionsbildes mit dem dritten Subtraktionsbild. Das kombinierte Subtraktionsbild wird zusammen mit dem ersten Subtraktionsbild zum Erzeugen des neuen Roadmap-Bildes verwendet. Wird der Parameterwert unmittelbar nach dem Aufnehmen des ersten Untersuchungsbildes verändert, so kann das Hilfssubtraktionsbild dem zweiten Subtraktionsbild entsprechen. Bevorzugt ist es jedoch vorgesehen, dass nach dem Aufnehmen des ersten Untersuchungsbildes und vor dem Verändern des Parameterwertes wenigstens ein, insbesondere mehrere, weitere Untersuchungsbildern des Zielobjekts aufgenommen werden.

Dadurch, dass das Hilfssubtraktionsbild aus dem zuletzt aufgenommenen dieser weiteren Untersuchungsbilder erzeugt wird, enthält das Hilfssubtraktionsbild also insbesondere einen gesamten bis zum Verändern des Parameterwertes aufgebauten Bildinhalt, beispielsweise hinsichtlich einer Ausbreitung oder Verteilung eines Kontrastmittels und/oder einer Ausbreitung oder Positionierung eines sonstigen Materials, wie beispielsweise eines zumindest teilweise röntgen-opaken Klebers oder dergleichen. Dadurch, dass das Hilfssubtraktionsbild zum Erzeugen des neuen Roadmap-Bildes verwendet wird, kann vorteilhaft also erreicht werden, dass der bis zum Verändern des Parameterwertes aufgebaute Bildinhalt in dem neuen Roadmap-Bild enthalten, also erkennbar ist. Ohne die Verwendung oder Einbeziehung des Hilfssubtraktionsbildes in das neue Roadmap-Bild würde dieser bis zum Verändern des Parameterwertes aufgebaute Bildinhalt zumindest teilweise verloren gehen, da er als Teil des dritten Maskenbildes subtrahiert wird.

Durch diese Ausgestaltung des erfindungsgemäßen Verfahrens wird besonders vorteilhaft also die Veränderung des Parameterwertes, das heißt eine Anwendung entsprechender Funktionen des bildgebenden Geräts, während des Roadmap-Verfahrens mit entsprechendem Informationsgewinn, zumindest aber unter Erhaltung des gesamten aufgebauten oder gewonnenen Bildinhaltes ermöglicht. Somit ist also eine zeitliche Entwicklung des Zielobjekts oder in dem Zielobjekt für den jeweiligen Nutzer oder Betrachter kontinuierlich von einem Zeitpunkt vor dem Verändern des Parameterwertes bis zu einem Zeitpunkt nach dem Verändern des Parameterwertes, also über die Veränderung des Parameterwertes hinweg, konsistent nachvollziehbar und nachverfolgbar anhand des jeweils zuletzt erzeugten neuen Roadmap-Bildes.

Dies stellt einen bedeutenden Fortschritt gegenüber dem bisherigen Stand der Technik dar. Durch die vorliegende Erfindung kann eine wesentliche Erweiterung und Qualitätssteigerung einer heutzutage gegebenen Funktionalität entsprechender Bildgebungsverfahren, -geräte oder -anlagen bei Verwendung des Roadmap-Verfahrens erreicht werden. Dies gilt insbesondere bei Verwendung oder Nutzung entsprechender Funktionen, welche mit der Veränderung des oder eines entsprechenden Parameterwertes einhergehen. Effektiv wird somit erst durch die vorliegende Erfindung die Nutzung dieser Funktionen oder Funktionalitäten in Verbindung mit dem, also während des Roadmap-Verfahrens sinnvoll, das heißt mit einer für praktische Anwendungen ausreichenden Bildqualität, ermöglicht. Letztlich können somit Untersuchungen des Zielobjekts schneller und mit weniger Belastung für das Zielobjekt durchgeführt werden.

In vorteilhafter Weiterbildung der vorliegenden Erfindung wird durch das bildgebende Gerät auf einen Empfang eines entsprechenden Steuerbefehls zum Verändern des Parameterwertes hin vor dessen Ausführung, also bevor der Parameterwert tatsächlich verändert wird, automatisch ein Hilfsuntersuchungsbild des Zielobjekts aufgenommen. Dieses Hilfsuntersuchungsbild wird dann als das, insbesondere zuletzt oder unmittelbar, vor dem Verändern des Parameterwertes aufgenommene Untersuchungsbild zum Erzeugen des Hilfssubtraktionsbildes verwendet. Auf diese Weise wird vorteilhaft automatisch sichergestellt, dass in dem Hilfssubtraktionsbild tatsächlich ein gesamter bis zum Verändern des Parameterwertes aufgebauter Bildinhalt enthalten ist, da ein zeitlicher Abstand von dem Aufnehmen des zum Erzeugen des Hilfssubtraktionsbildes verwendeten Untersuchungsbildes - hier also des Hilfsuntersuchungsbild des - zu dem physischen Verändern des Parameterwertes minimiert werden kann. Dadurch kann vorteilhaft besonders zuverlässig und genau eine Kontinuität und Konsistenz des in dem neuen Roadmap-Bild dargestellten oder enthaltenen Bildinhaltes erreicht beziehungsweise erreicht werden. Zudem wird der jeweilige Benutzer entlastet, da er nicht selbst manuell zwei Funktionen oder Bedienhandlungen, nämlich das Aufnehmen des Hilfsuntersuchungsbildes und das Verändern des Parameterwertes, möglichst zeitnah zueinander auslösen oder durchführen muss. Dies kann letztlich zu einem verbesserten oder zuverlässigeren Ergebnis einer Untersuchung oder Bewertung des Zielobjekts beitragen und eine Fehlerrate bei der Bedienung des bildgebenden Geräts verringern.

In vorteilhafter Weiterbildung des erfindungsgemäßen Verfahrens werden das Hilfssubtraktionsbild und das kombinierte Subtraktionsbild als jeweils mittelwertfreie Bilder erzeugt, sodass ein jeweiliger Mittelwert von deren Pixel- oder Intensitätswerten Null oder zumindest nahezu Null ist. Mit anderen Worten werden das Hilfssubtraktionsbild und das kombinierte Subtraktionsbild also so erzeugt, dass deren Pixelwerte um Null herum verteilt sind, wobei selbstverständlich nicht alle Pixelwerte jeweils für sich genommen gleich Null sind. Hierdurch kann vorteilhaft eine besonders einfache Weiterverarbeitung dieser Bilder und letztlich eine besonders gute Bildqualität des neuen Roadmap-Bildes erreicht werden. Bevorzugt wird auch das wenigstens eine dritte Subtraktionsbild in entsprechender Weise als mittelwertfreies Bild erzeugt.

In vorteilhafter Weiterbildung der vorliegenden Erfindung wird zum Erzeugen des kombinierten Subtraktionsbildes das dritte Subtraktionsbild zu dem Hilfssubtraktionsbild addiert. Das bedeutet, dass die Pixel- oder Intensitätswerte des dritten Subtraktionsbildes und des Hilfssubtraktionsbildes pixelweise aufaddiert werden und eine entsprechende Summe als Pixel- oder Intensitätswert für einen jeweiligen entsprechenden Pixel des kombinierten Subtraktionsbildes verwendet wird. Hier bedeutet das Kombinieren des Hilfssubtraktionsbildes mit dem dritten Subtraktionsbildes also deren Addition. So kann das kombinierte Subtraktionsbild besonders schnell und einfach erzeugt werden, was eine Echtzeit-Anwendung des erfindungsgemäßen Verfahrens erleichtert.

Dabei ist es - wie bei allen Überlagerungen oder Kombinationen zweier Bilder im Sinne der vorliegenden Erfindung - vorgesehen, dass jeweils zwei einander überlagerte oder miteinander kombinierte Bilder miteinander oder zueinander registriert sind oder werden. Das bedeutet, dass mit einander überlagerte oder kombinierte Pixel zweier Bilder jeweils den gleichen Teilbereich des Zielobjekts darstellen oder abbilden. Bei Bedarf, beispielsweise wenn sich das bildgebende Gerät und/oder das Zielobjekt zwischen dem Aufnehmen der jeweiligen zwei miteinander überlagerten oder kombinierten Bilder bewegt hat, kann dementsprechend eine Registrierung oder Neu-Registrierung, insbesondere automatisch, durchgeführt werden.

In vorteilhafter Weiterbildung der vorliegenden Erfindung werden zum Erzeugen des kombinierten Subtraktionsbildes vor dem Kombinieren des Hilfssubtraktionsbildes mit dem dritten Subtraktionsbild automatisch Pixelwerte des Hilfssubtraktionsbildes mit einem von 1 verschiedenen vorgegebenen Faktor multipliziert. Ist dieser vorgegebene Faktor kleiner als 1, wird also letztlich ein Bildanteil des neuen Roadmap-Bildes, welcher das Zielobjekt oder dessen Zustand vor dem Verändern des Parameterwertes repräsentiert, abgeschwächt dargestellt gegenüber einem Bildanteil des Roadmap-Bildes, welcher das Zielobjekt oder dessen Zustand nach dem Verändern des Parameterwertes repräsentiert. Dadurch kann vorteilhaft ein Effekt der Veränderung des Parameterwertes besonders einfach erkennbar gemacht werden. Zudem kann so vorteilhaft auf besonders einfache Weise eine zeitliche Entwicklung in dem einen neuen Roadmap-Bild veranschaulicht werden. Wird der vorgegebene Faktor auf 0 gesetzt, entspricht dies einer vollständigen Ausblendung des bis zum Verändern des Parameterwertes aufgebauten Bildinhaltes. Der Faktor kann beispielsweise je nach Anwendung, Situation, Bedarf oder Kundenwunsch vorgegeben sein oder werden. Bevorzugt ist der Faktor beziehungsweise dessen Wert über eine Benutzerschnittstelle des bildgebenden Geräts einstellbar oder vorgebbar sein. Hierdurch wird vorteilhaft eine besonders flexible Nutzung des bildgebenden Geräts und des erfindungsgemäßen Verfahrens ermöglicht.

In vorteilhafter Weiterbildung der vorliegenden Erfindung wird zum Erzeugen des kombinierten Subtraktionsbildes ein Farbwert oder eine Farbdarstellung des Hilfssubtraktionsbildes und/oder des dritten Subtraktionsbildes automatisch verändert, sodass ein aus dem Hilfssubtraktionsbild stammender Anteil des kombinierten Subtraktionsbildes eine andere Darstellungsfarbe aufweist als ein aus dem dritten Subtraktionsbild stammender Anteil des kombinierten Subtraktionsbildes. Mit anderen Worten wird also der zeitliche Verlauf oder eine zeitliche Veränderung und somit insbesondere ein Effekt der Veränderung des Parameterwertes durch eine entsprechende anteilsweise oder bereichsweise Farbkodierung repräsentiert oder veranschaulicht. Dies ermöglicht eine besonders einfache und anschaulich nachvollziehbare Erkennbarkeit oder Unterscheidbarkeit von Bildinhalten, die verschiedene Zeitpunkte oder Zustände oder Aufnahmebedingungen repräsentieren.

In vorteilhafter Weiterbildung der vorliegenden Erfindung wird zum Erzeugen des kombinierten Subtraktionsbildes vor dem Kombinieren des Hilfssubtraktionsbildes mit dem dritten Subtraktionsbild automatisch durch einen Vergleich des Hilfssubtraktionsbildes mit dem dritten Subtraktionsbild ein Bildbereich bestimmt, in welchem das dritte Subtraktionsbild einen in dem Hilfssubtraktionsbild nicht vorhandenen oder einen gegenüber diesem veränderten Bildinhalt aufweist. In diesem bestimmten Bildbereich trägt das dritte Subtraktionsbild also zusätzliche oder neue Bildinformationen, nämlich den entsprechenden Bildinhalt, zu dem kombinierten Hilfssubtraktionsbild bei. Die bestimmte Bildbereich kann also insbesondere ein Teilbereich des dritten Subtraktionsbildes sein. Da die Bilder jedoch ohnehin pixelgenau überlagert werden können, entspricht dieser Bildbereich damit hinsichtlich seiner Anordnung und Größe automatisch auch einem entsprechenden Bild- oder Teilbereich des Hilfssubtraktionsbildes und des kombinierten Subtraktionsbildes.

In dieser Ausgestaltung der Erfindung wird dann auf einen dem bestimmten Bildbereich entsprechenden Teilbereich des Hilfssubtraktionsbildes ein vorgegebener Schwellenwertfilter zur Rauschunterdrückung angewendet. Mit anderen Worten werden also vor dem Kombinieren des Hilfssubtraktionsbildes mit dem dritten Subtraktionsbild automatisch durch Anwendung eines Schwellenwertverfahrens gerade in demjenigen Bildbereich oder in denjenigen Bild- oder Teilbereichen des Hilfssubtraktionsbildes das Rauschen aus dem Hilfssubtraktionsbild entfernt, in denen der in dem Hilfssubtraktionsbild nicht vorhandene, also neue oder zusätzliche, von dem dritten Subtraktionsbild beigesteuerte Bildinhalt vorhanden ist. Auf diese Weise kann gezielt dieser neue oder zusätzliche Bildinhalt besonders klar und rauscharm abgebildet oder dargestellt werden. Gleichzeitig gehen bei der Anwendung des Schwellenwertfilters in den übrigen oder restlichen Bildbereichen keine Informationen verloren, da hier der Schwellenwertfilter nicht angewendet wird. Insbesondere können durch den Schwellenwertfilter beispielsweise alle Pixel- oder Intensitätswerte des Hilfssubtraktionsbildes in dem bestimmten Bildbereich auf 0 gesetzt werden. Jedenfalls ermöglicht diese Ausgestaltung des erfindungsgemäßen Verfahrens eine situationsspezifische, also an den jeweiligen Einzelfall angepasste optimierte Bildqualität.

In vorteilhafter Weiterbildung der vorliegenden Erfindung werden durch das bildgebende Gerät nach einem Empfangen eines entsprechenden Steuerbefehls zum Verändern des Parameterwertes vor dessen Ausführung, also vor dem tatsächlichen Verändern des Parameterwertes, automatisch mehrere Hilfsuntersuchungsbilder des Zielobjekts aufgenommen. Diese mehreren Hilfsuntersuchungsbilder werden dann automatisch gemittelt, um ein Rauschen in dem Hilfssubtraktionsbild zu reduzieren. Dazu kann ein durch das Mitteln der mehreren Hilfsuntersuchungsbilder erzeugtes gemitteltes Untersuchungsbild als das zum Erzeugen des Hilfssubtraktionsbildes verwendete Untersuchungsbild verwendet werden. Dadurch, dass die mehreren Hilfsuntersuchungsbilder automatisch und erst nach Empfang des Steuerbefehls zum Verändern des Parameterwertes aufgenommen werden, kann so auf besonders einfache und zuverlässige Art und Weise das Rauschen in dem resultierenden Hilfssubtraktionsbild reduziert werden, ohne dass bis zu diesem Zeitpunkt bereits aufgebauter Bildinhalt verloren geht oder eine aufwändige Registrierung notwendig wäre. Somit kann letztlich insgesamt auf besonders einfache Weise eine verbesserte Bildqualität erreicht werden.

In vorteilhafter Weiterbildung der vorliegenden Erfindung wird zum Aufnehmen der mehreren Hilfsuntersuchungsbilder automatisch eine Bildaufnahmefrequenz oder Pulsrate des bildgebenden Geräts gegenüber einem bis dahin verwendeten entsprechenden Wert erhöht. Mit anderen Worten werden also die mehreren Hilfsuntersuchungsbilder schneller beziehungsweise mit kürzerem zeitlichem Abstand zueinander aufgenommen als die bis dahin aufgenommenen Bilder. Dies ist aus zumindest zweierlei Gründen besonders vorteilhaft. Zum einen kann so erreicht werden, dass die mehreren Hilfsuntersuchungsbilder einen möglichst gleichen Zustand des Zielobjekts abbilden. Während es für die vorherigen und nachfolgenden Aufnahmen oder Bilder sinnvoll sein kann, zwischen jeweils zwei Bildern oder Aufnahmen eine vorgegebene minimale Zeitdauer verstreichen zu lassen, um beispielsweise eine weitere Ausbreitung eines Kontrastmittels oder dergleichen abzuwarten, ist dies für die mehreren Hilfsuntersuchungsbilder nicht notwendig, da diese hier lediglich als zusätzliche Datenbasis für die Rauschreduktion dienen. Eine möglichst große Bildaufnahmefrequenz ist daher besonders vorteilhaft für die Aufnahme der mehreren Hilfsuntersuchungsbilder.

Zum anderen kann durch die erhöhte Bildaufnahmefrequenz eine Verzögerung zwischen dem Geben beziehungsweise Empfangen des Steuerbefehls zum Verändern des Parameterwertes und dessen Ausführung oder Umsetzung reduziert werden. Wird als Parameter beispielsweise ein Zoom oder eine Zoomstufe verwendet, so kann durch die erhöhte Bildaufnahmefrequenz oder Pulsrate somit eine Übergangszeit zu einer gezoomten Darstellung verkürzt werden. Zusätzlich zu den mehreren Hilfsuntersuchungsbildern kann dabei ebenso das zuvor erwähnte erste Untersuchungsbild in entsprechender Weise verwendet werden.

Besonders bevorzugt kann es vorgesehen sein, dass die Bildaufnahmefrequenz oder Pulsrate durch das bildgebende Gerät nach dem Aufnehmen der mehreren Hilfsuntersuchungsbilder automatisch wieder auf ihren vorherigen Wert gesetzt, also entsprechend reduziert wird. Dadurch kann eine unnötige Belastung des bildgebenden Geräts und/oder des Zielobjekts vermieden werden.

Ein weiterer Aspekt der vorliegenden Erfindung ist ein Datenspeicher oder Datenträger mit einem darin gespeicherten Programmcode, welcher die Verfahrensschritte zumindest einer Ausführungsform des erfindungsgemäßen Verfahrens zum Erzeugen eines Roadmap-Bildes kodiert oder repräsentiert. Dieser Programmcode ist dabei ausgebildet mittels einer Prozessoreinrichtung zum Durchführen des entsprechenden kodierten Verfahrens ausgeführt zu werden.

Ein weiterer Aspekt der vorliegenden Erfindung ist ein bildgebendes Gerät, insbesondere ein medizinisches bildgebendes Gerät, umfassend eine Datenverarbeitungseinrichtung zum automatischen Verarbeiten von mittels des bildgebenden Geräts aufgenommenen Bildern eines Zielobjekts. Die Datenverarbeitungseinrichtung umfasst dabei einen erfindungsgemäßen Datenspeicher und eine mit diesem verbundene Prozessoreinrichtung zum Ausführen des in dem Datenspeicher gespeicherten Programmcodes. Mit anderen Worten ist das erfindungsgemäße bildgebende Gerät also zum Durchführen oder Ausführen zumindest einer Ausführungsform des erfindungsgemäßen Verfahrens eingerichtet. Dementsprechend kann das erfindungsgemäße bildgebende Gerät insbesondere das im Zusammenhang mit dem erfindungsgemäßen Verfahren genannte bildgebende Gerät sein. Somit kann das erfindungsgemäße bildgebende Gerät entsprechend insbesondere die im Zusammenhang mit dem erfindungsgemäßen Verfahren beschriebenen Eigenschaften und/oder Komponenten aufweisen. Dies kann beispielsweise die genannte Benutzerschnittstelle betreffen. Das erfindungsgemäße bildgebende Gerät kann insbesondere ein Röntgengerät oder Fluoroskopiegerät sein.

Die bisher und im Folgenden angegebenen Eigenschaften und Weiterbildungen des erfindungsgemäßen Verfahrens sowie die entsprechenden Vorteile sind jeweils sinngemäß auf die anderen Aspekte der Erfindung, also auf den erfindungsgemäßen Datenspeicher und das erfindungsgemäße bildgebende Gerät, und/oder zur Durchführung des erfindungsgemäßen Verfahrens verwendete oder verwendbare Bauteile und Einrichtungen übertragbar und umgekehrt. Es gehören also zu der Erfindung auch solche Weiterbildungen des erfindungsgemäßen Verfahrens, des erfindungsgemäßen Datenspeichers und des erfindungsgemäßen bildgebenden Geräts, welche Ausgestaltungen aufweisen, die hier zur Vermeidung unnötiger Redundanz nicht explizit in der jeweiligen Kombination für alle Aspekte der Erfindung separat beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:

FIG 1     eine schematische Übersichtsdarstellung zur Veranschaulichung eines Verfahrens zum Erzeugen eines Bildes eines Zielobjekts in einer ersten Variante;

FIG 2     eine schematische Übersichtsdarstellung zur Veranschaulichung eines Verfahrens zum Erzeugen eines Bildes eines Zielobjekts in einer zweiten Variante; und

FIG 3     eine schematische Darstellung eines erzeugten Bildes eines Zielobjekts.

Bei den im Folgenden erläuterten Ausführungsbeispielen handelt es sich um bevorzugte Ausführungsformen der Erfindung.

Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind gleiche oder einander entsprechende Elemente jeweils mit den gleichen Bezugszeichen gekennzeichnet.

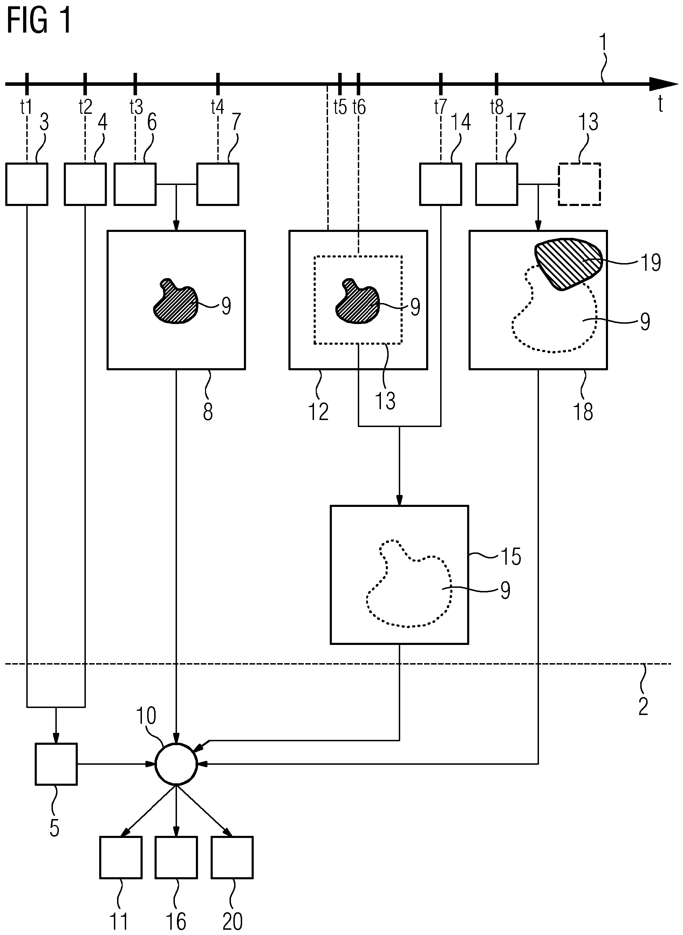

FIG 1 zeigt eine schematische Übersichtsdarstellung zur Veranschaulichung einer ersten Variante eines Verfahrens zum Erzeugen eines Bildes eines Zielobjekts unter Anwendung eines Roadmap-Verfahrens. In dieser Übersichtsdarstellung ist eine Zeitachse 1 dargestellt. Auf dieser Zeitachse 1 sind mehrere Zeitpunkte t1 bis t8 markiert, zu denen verschiedene Verfahrensschritte des Verfahrens ausgeführt werden. Der Zeitpunkt t1 stellt dabei den frühesten Zeitpunkt und der Zeitpunkt t8 den spätesten Zeitpunkt dar.

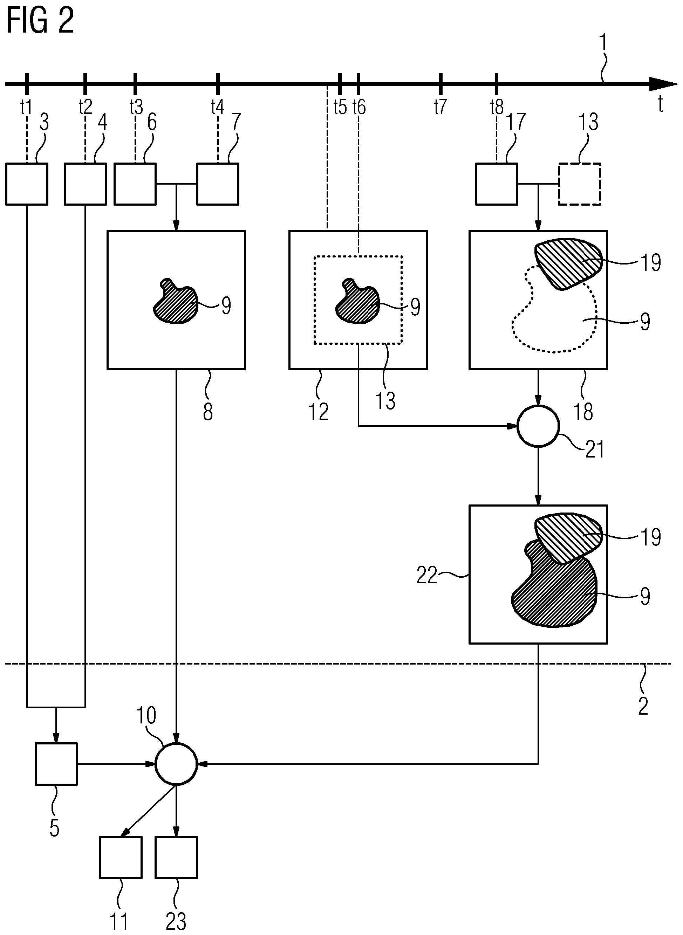

Eine zeitliche Übereinstimmung oder Zuordnung von dargestellten Elementen mit der Zeitachse 1 gilt nur für zwischen der Zeitachse 1 und einer Hilfslinie 2 dargestellte Elemente. Insbesondere unterhalb, also auf einer von der Zeitachse 1 abgewandten Seite der Hilfslinie 2 dargestellte Elemente können gegebenenfalls zu anderen Zeitpunkten ausgeführt werden oder relevant sein als ihre Anordnung in Bezug zu der Zeitachse 1 andeuten würde. Dies gilt ebenso für die weiter unten erläuterte FIG 2 in entsprechender Weise.

Zum Zeitpunkt t1 wird mittels eines medizinischen bildgebenden Geräts, insbesondere mittels eines Röntgengeräts, zunächst ein erstes Maskenbild 3 des Zielobjekts aufgenommen. Das Zielobjekt kann dabei beispielsweise ein Patient oder ein bestimmter Teilbereich des Patienten sein. Das erste Maskenbild 3 wird insbesondere aufgenommen bevor ein Kontrastmittel oder dergleichen in einen Aufnahmebereich des Bildgeräts beziehungsweise in das Zielobjekt eingebracht wird. Nach Aufnahme des ersten Maskenbildes 3 kann dann beispielsweise das Kontrastmittel in das Zielobjekt eingebracht werden und sich in diesem ausbreiten. Zu einem Zeitpunkt t2 wird dann ein Injektions- oder Kontrastmittelbild 4 des Zielobjekts aufgenommen. Das erste Maskenbild 3 und das Kontrastmittelbild 4 werden dabei mit denselben röntgen-physikalischen Parametern oder Einstellungen des bildgebenden Geräts aufgenommen.

Nachdem das erste Maskenbild 3 und das Kontrastmittelbild 4 aufgenommen worden sind, wird aus diesen ein erstes Subtraktionsbild 5 erzeugt, indem das erste Maskenbild 3 von dem Kontrastmittelbild 4 subtrahiert wird. Im vorliegenden Beispiel handelt es sich bei dem ersten Subtraktionsbild 5 um ein Gefäßbild (englisch: "Vessel map"), also beispielsweise ein Bild eines Gefäßbaumes.

Zu einem Zeitpunkt t3 wird ein zweites Maskenbild 6 und zu einem Zeitpunkt t4 ein erstes Untersuchungsbild 7 des Zielobjekts aufgenommen. Auch das zweite Maskenbild 6 und das erste Untersuchungsbild 7 werden dabei mit denselben röntgen-physikalischen Parametern oder Parameterwerten des bildgebenden Geräts, also unter denselben Aufnahmebedingungen aufgenommen. Diese Parameterwerte oder Aufnahmebedingungen können die gleichen sein wie die für das erste Maskenbild 3 und das Kontrastmittelbild 4 verwendeten Parameterwerte beziehungsweise Einstellungen oder Aufnahmebedingungen. Ebenso können jedoch für das erste Maskenbild 3 und das Kontrastmittelbild 4 einerseits sowie für das zweite Maskenbild 6 und das erste Untersuchungsbild 7 andererseits jeweils unterschiedliche Parameterwerte oder Einstellungen, also Aufnahmebedingungen verwendet werden. So ist insgesamt sichergestellt, dass aus den jeweils zwei Bildern artefaktfrei ein entsprechendes Subtraktionsbild erzeugt werden kann.

Aus dem ersten Untersuchungsbild 7 wird durch Subtraktion des zweiten Maskenbilds 6 ein zweites Subtraktionsbild 8 erzeugt. Das zweite Subtraktionsbild 8 zeigt hier einen ersten Bildinhalt 9, der sich zwischen den Zeitpunkten t3 und t4 aufgebaut hat. Wird das Verfahren beispielsweise angewendet während in einer interventionellen Prozedur eine arteriovenöse Malformation (AVM) mittels eines radio- oder röntgen-opaken Klebers verklebt wird, so kann das zweite Subtraktionsbild 8 beziehungsweise der erste Bildinhalt 9 beispielsweise eine nach dem Zeitpunkt t3 in das Zielobjekt beziehungsweise in den Aufnahmebereich eingebrachte Verteilung dieses Klebers darstellen. Grundsätzlich kann das zweite Subtraktionsbild 8 ebenso zu einem späteren als dem hier dargestellten Zeitpunkt erzeugt werden.

Das erste Subtraktionsbild 5 und das zweite Subtraktionsbild 8 werden dann durch Anwendung einer Kombinationsfunktion 10 miteinander kombiniert also etwa einander überlagert. Hierdurch wird ein erstes Roadmap-Bild 11 erzeugt, welches als Doppelsubtraktionsbild sowohl das Gefäßbild als auch den ersten Bildinhalt 9, also etwa den Kleber und/oder andere Miniaturwerkzeuge, wie etwa einen Stent, einen Katheter oder dergleichen, zeigt. Entsprechend kann das zweite Subtraktionsbild 8 auch als Geräte- oder Device-Bild bezeichnet werden. Vorliegend wird davon ausgegangen, dass hierbei lediglich 2D-Bilder verarbeitet beziehungsweise miteinander kombiniert oder überlagert werden.

Das Erzeugen der Subtraktionsbilder 5, 8 kann also ausgedrückt werden als

$$VM = P(t2)-P(t1) \text{ und } DV(t4) = P(t4)-P(t3) \quad ,$$

wobei VM das erste Subtraktionsbild 5, DV(t4) das aus dem zum Zeitpunkt t4 aufgenommenen ersten Untersuchungsbild 7 erzeugte zweite Subtraktionsbild 8 und P(tx) mit x E [1,8] jeweils das zum Zeitpunkt tx aufgenommene Bild bezeichnen. Das Erzeugen des ersten Roadmap-Bildes 11 kann dementsprechend ausgedrückt werden als

$$RDMP(tx) = K[VM, DV(tx)] ,$$

wobei RDMP(tx) das Roadmap-Bild, welches aus einem zum Zeitpunkt tx aufgenommenen Untersuchungsbild erzeugt wird und K[] die Kombinationsfunktion mit den Argumenten VM und DV(t). Durch die Kombinationsfunktion K[] werden in diesem Fall also die Bilder VM und DV(t) zum Erzeugen des Roadmap-Bildes RDMP(tx) miteinander kombiniert.

Im vorliegenden Beispiel ist es vorgesehen, dass zu einem Zeitpunkt t5 eine zum Aufnehmen der Bilder 3, 4, 6, 7 verwendete Zoomstufe des bildgebenden Geräts verändert wird. Die Zoomstufe ist dabei ein röntgen-physikalischer Parameter des bildgebenden Geräts, deren Wert zu einer Veränderung der Aufnahmebedingungen führt. Nach dem Zeitpunkt t5 aufgenommene Bilder können somit nicht ohne weiteres, insbesondere nicht ohne Entstehung von störenden Bildartefakten, mit vor dem Zeitpunkt t5 aufgenommenen Bildern kombiniert werden, um ein entsprechendes Subtraktionsbild zu erzeugen. Aus diesem Grund wird vorliegend unmittelbar vor dem Zeitpunkt t5 ein Hilfssubtraktionsbild 12 erzeugt. Dazu wird von einem hier der Übersichtlichkeit halber nicht einzeln dargestellten Untersuchungsbild des Zielobjekts das zweite Maskenbild 3 subtrahiert. Das hierfür verwendete Untersuchungsbild ist dabei das im jeweiligen Einzelfall nach dem Zeitpunkt t3 und zuletzt vor dem Zeitpunkt t5 aufgenommene Untersuchungsbild des Zielobjekts. Wird zwischen den Zeitpunkten t4 und t5 kein weiteres Untersuchungsbild des Zielobjekts aufgenommen, so kann hier das erste Untersuchungsbild 7 verwendet werden, um das Hilfssubtraktionsbild 12 zu erzeugen.

Anschließend wird zu dem Zeitpunkt t5 die Zoomstufe verändert. Dazu wird vorliegend die Zoomstufe erhöht, sodass ein kleinerer Bildausschnitt, also ein kleinerer Teilbereich des Zielobjekts aufgenommen oder abgebildet wird. Unmittelbar nach dem Verändern der Zoomstufe wird zu einem Zeitpunkt t6 automatisch ein drittes Maskenbild 13 aufgenommen. Es ist hier zu erkennen, dass das dritte Maskenbild 13 einen kleineren Teilbereich des Zielobjekts abbildet als etwa das Hilfssubtraktionsbild 12 und das zweite Subtraktionsbild 8. Insbesondere ist die Zoomstufe für das dritte Maskenbild 13 hier in Abhängigkeit von dem ersten Bildinhalt 9 beziehungsweise in Abhängigkeit von dessen Größe gewählt, sodass der erste Bildinhalt 9 einen Großteil des dritten Maskenbildes 13 einnimmt. Hierdurch kann in dieser veränderten Zoomstufe eine verbesserte Detaildarstellung des ersten Bildinhaltes 9 und von dessen unmittelbarer Umgebung erzielt werden. Das dritte Maskenbild 13 enthält dementsprechend in dem aufgenommenen Bildbereich den gesamten bis zum Zeitpunkt t5 beziehungsweise t6 aufgebauten Bildinhalt, insbesondere den ersten Bildinhalt 9. Es ist dabei vorgesehen, dass die Zeitpunkte t5 und t6 zeitlich so nahe beieinander liegen, dass sich zwischen diesen zumindest im Wesentlichen kein zusätzlicher oder neuer weiterer Bildinhalt aufbaut.

Zu einem Zeitpunkt t7 wird ein zweites Untersuchungsbild 14 des Zielobjekts aufgenommen. Aus diesem zweiten Untersuchungsbild 14 wird das dritte Maskenbild 13 subtrahiert, um ein drittes Subtraktionsbild 15 zu erzeugen. In dem dritten Subtraktionsbild 15 ist der erste Bildinhalt 9 nicht mehr enthalten und daher hier gestrichelt angedeutet. Wird nun durch Anwendung der Kombinationsfunktion 10 auf das erste Subtraktionsbild 5 und das dritte Subtraktionsbild 15 ein zweites Roadmap-Bild 16 erzeugt, so ist in diesem daher der bis zum Zeitpunkt t5 beziehungsweise t6 aufgebaute Bildinhalt, insbesondere der erste Bildinhalt 9, nicht mehr erkennbar.

Zu einem Zeitpunkt t8 wird ein drittes Untersuchungsbild 17 des Zielobjekts aufgenommen. Aus diesem wird durch Subtraktion des dritten Maskenbildes 13 ein viertes Subtraktionsbild 18 erzeugt. Damit ist auch in dem vierten Subtraktionsbild 18 also der erste Bildinhalt 9 nicht enthalten und daher auch hier nur zur besseren Orientierung nur gestrichelt angedeutet. Da zwischen den Zeitpunkten t6 und t8 jedoch einige Zeit verstrichen ist, hat sich seit dem Zeitpunkt t6, also seit dem Aufnehmen des dritten Maskenbildes 13 bis zum Zeitpunkt t8 nunmehr weiterer Bildinhalt, hier nämlich ein zweiter Bildinhalt 19, aufgebaut, der somit in dem dritten Untersuchungsbild 17 aber nicht in dem dritten Maskenbild 13 enthalten ist und daher in dem vierten Subtraktionsbild 18 erkennbar bleibt. Im vorliegenden Beispiel wurde zwischen den Zeitpunkten t7 und t8 neuer radio- beziehungsweise röntgen-opaker Kleber eingebracht, der nun in Form des zweiten Bildinhaltes 19 in dem vierten Subtraktionsbild 18 erkennbar ist.

Durch Anwendung der Kombinationsfunktion 10 auf das erste Subtraktionsbild 5 und das vierte Subtraktionsbild 18 kann nunmehr ein drittes Roadmap-Bild 20 erzeugt werden. Aus einer Zusammenschau der Roadmap-Bilder 11, 16, 20 kann somit eine zeitliche Entwicklung der jeweiligen interventionellen Prozedur nachvollzogen werden.

FIG 2 zeigt eine schematische Übersichtsdarstellung zur Veranschaulichung einer zweiten Variante des Verfahrens. Die in FIG 2 gezeigte Übersichtsdarstellung weist grundsätzlich die gleiche Struktur wie die in FIG 1 gezeigte Übersichtsdarstellung auf. Es werden daher lediglich die Unterschiede der in FIG 2 veranschaulichten zweiten Variante des Verfahrens gegenüber der in FIG 1 veranschaulichten ersten Variante des Verfahrens erläutert.

In der in FIG 2 veranschaulichten zweiten Variante des Verfahrens werden das vierte Subtraktionsbild 18 und das vor dem Verändern der Zoomstufe aufgenommene beziehungsweise erzeugte Hilfssubtraktionsbild 12 miteinander kombiniert. Dazu wird auf das Hilfssubtraktionsbild 12 und das vierte Subtraktionsbild 18 eine Überlagerungsfunktion 21 angewendet. Je nach Bedarf werden dabei das Hilfssubtraktionsbild 12 und das vierte Subtraktionsbild 18 miteinander addiert, mit einem oder mehreren vorgegebenen oder eingestellten Faktoren multipliziert, in ihren Farbwerten angepasst und/oder zumindest bereichsweise einem Schwellenwertverfahren zur Rauschunterdrückung unterworfen. Im Ergebnis ergibt sich durch die Anwendung der Überlagerungsfunktion 21 ein kombiniertes Subtraktionsbild 22. Zu diesem kombinierten Subtraktionsbild 22 haben also insgesamt das zweite Maskenbild 6, das zuletzt vor dem Zeitpunkt t5 aufgenommene Untersuchungsbild, das Maskenbild 13 und das dritte Untersuchungsbild 17 beigetragen. Daher sind in dem kombinierten Subtraktionsbild 22 sowohl der erste Bildinhalt 9 als auch der zweite Bildinhalt 19 enthalten, also erkennbar. Die Erzeugung des kombinierten Subtraktionsbildes 22 kann also ausgedrückt werden als

$$DVK = F[DV(t5), DV(t8)],$$

wobei DVK(tx) das kombinierte Subtraktionsbild, F[] die Überlagerungsfunktion 21, DV(t5) das Hilfssubtraktionsbild 12 und DV(t8) das vierte Subtraktionsbild 18 bezeichnen.

Anschließend werden das erste Subtraktionsbild 5 und das kombinierte Subtraktionsbild 22 durch Anwendung der Kombinationsfunktion 10 miteinander kombiniert und somit ein viertes Roadmap-Bild 23 erzeugt. Das Erzeugen des vierten Roadmap-Bildes 23 kann also entsprechend der obigen Notation ausgedrückt werden als

$$RDMP(t8) = K[VM, F[DV(t5), DV(t8)]] \quad .$$

In diesem vierten Roadmap-Bild 23 ist vorteilhaft nunmehr der gesamte zwischen den Zeitpunkten t3 und t8 aufgebaute Bildinhalt 9, 19 enthalten und erkennbar. Damit ist also allein anhand des vierten Roadmap-Bildes 23 die zeitliche Entwicklung der jeweiligen interventionellen Prozedur ohne Bildartefakte über die Veränderung der Zoomstufe zum Zeitpunkt t5 hinweg nachvollziehbar.

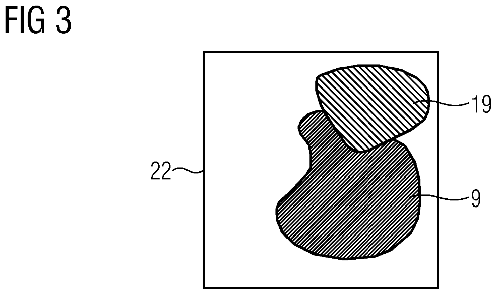

FIG 3 zeigt eine schematische Darstellung des kombinierten Subtraktionsbildes 22 für den Fall, dass als Teil der Überlagerungsfunktion 21 ein Schwellenwertverfahren (Thresholding) und/oder eine Farbwertanpassung für den ersten Bildinhalt 9 und/oder den zweiten Bildinhalt 19 beziehungsweise die entsprechenden Bildbereiche angewendet wurde. Durch diese Maßnahmen kann der erste Bildinhalt 9 von dem zweiten Bildinhalt 19 besonders einfach unterscheidbar in dem kombinierten Subtraktionsbild 22 und somit letztlich auch in dem vierten Roadmap-Bild 23 dargestellt werden. Somit kann aus dem Roadmap-Bild 23 nicht nur der gesamte zwischen den Zeitpunkten t3 und t8 aufgebaute Bildinhalt 9, 19 abgelesen werden, sondern auch dessen zeitliche Entwicklung anhand der unterschiedlichen Darstellungen besonders einfach nachvollzogen werden.

In den Figuren sind beispielhaft nur die Bilder 8, 12, 13, 15, 18 und 22 mit einem entsprechenden Bildinhalt 9, 19 dargestellt, während die übrigen Bilder 3, 4, 5, 6, 7, 11, 14, 16, 17, 20 und 23 lediglich schematisch angedeutet sind. Diese schematisch angedeuteten Bilder können jedoch selbstverständlich jeweils die gleiche Größe wie die beispielhaft mit dem entsprechenden Bildinhalt 9, 19 dargestellten Bilder 8, 12, 13, 18 und 22 aufweisen und dasselbe Zielobjekt in entsprechender Weise darstellen oder abbilden. Die in den Figuren dargestellten Größen einzelner Elemente geben also keinen direkten Hinweis auf eine Größe entsprechender realer Gegenstücke bei einer konkreten Umsetzung oder Realisierung des beschriebenen Verfahrens.

Durch das beschriebene Verfahren kann vorteilhaft die Zoomfunktion oder eine andere, die Bildaufnahmebedingungen beeinflussende Funktion oder Funktionalität des bildgebenden Geräts, in dem Roadmap-Verfahren angewendet oder eingesetzt werden. Dabei kann die Anwendung der Zoomfunktion vorteilhaft eine verbesserte Erkennbarkeit der Bildinhalte 9, 19 mit sich bringen. Bei heutzutage verbreiteten bestehenden digitalen Systemen, also bildgebenden Geräten, ändert sich eine verwendete Pixelgröße unabhängig von der eingestellten Zoomstufe nicht. Für die verbesserte Erkennbarkeit wird bei der Veränderung der Zoomstufe das bildgebende Gerät beziehungsweise ein verwendeter Röntgenstrahlkegel auf ein kleineres Bildformat oder einen kleineren Bildausschnitt, also einen kleineren Teilbereich des Zielobjekts, kollimiert. Zudem kann eine Strahlungsdosis für eine bessere Erkennbarkeit erhöht werden. Durch das kleinere Bildformat wird jedoch die Gesamt- oder Hautdosisbelastung des Zielobjekts - also beziehungsweise des Patienten - nicht erhöht. Durch die Kollimierung verändern sich jedoch beispielsweise Streustrahlungsgegebenheiten. Dies wird in dem vorliegenden Verfahren dadurch berücksichtigt, dass zum Erzeugen der Subtraktionsbilder 15, 18 aus den nach dem Zeitpunkt t6 aufgenommenen Untersuchungsbildern 14, 17 das ebenfalls nach dem Zeitpunkt t5 aufgenommene dritte Maskenbild 13 verwendet wird. Dadurch wird vorteilhaft vermieden, dass die aus den Untersuchungsbildern 14, 17 erzeugten Subtraktionsbilder 15, 18 durch von der Veränderung der Zoomstufe zum Zeitpunkt t5 herrührende Bildartefakte praktisch unbrauchbar werden. Würde statt des dritten Maskenbildes 13 beispielsweise auch nach dem Zeitpunkt t5 weiterhin das zweite Maskenbild 6 verwendet, so ließe sich die Zoomfunktion effektiv nicht praktikabel anwenden, um einen Informationsgewinn im Bereich der Bildinhalte 9, 19 zu erzielen. Durch das automatische Aufnehmen und das Verwenden des dritten Maskenbilds 13 kann man sich bei dem Verfahren also physikalisch optimal auf die dann gegebenen neuen Aufnahmebedingungen einstellen.

**Patentansprüche**

1. Verfahren zum Erzeugen wenigstens eines neuen Roadmap-Bildes (16, 20, 23) eines mittels eines bildgebenden Geräts abgebildeten Zielobjekts, mit den Verfahrensschritten

   - Erzeugen eines ersten Roadmap-Bildes (11) durch Überlagerung eines ersten (5) und eines zweiten Subtraktionsbildes (9), wobei das erste Subtraktionsbild (5) erzeugt wird aus jeweils einem mittels des Geräts aufgenommenen ersten Maskenbild (3) und Kontrastmittelbild (4) des Zielobjekts, und das zweite Subtraktionsbild (9) erzeugt wird aus jeweils einem mittels des Geräts aufgenommenen zweiten Maskenbild (6) und ersten Untersuchungsbild (7) des Zielobjekts,

   **dadurch gekennzeichnet, dass**
   nach dem Erzeugen des ersten Roadmap-Bildes (11)

   - ein Parameterwert eines physikalischen Parameters des Geräts verändert wird (t5), welcher das Aufnehmen von Bildern des Zielobjekts mittels des Geräts beeinflusst,
   - unmittelbar nach dem Verändern des Parameterwertes unter Verwendung des veränderten Parameterwertes durch das Gerät automatisch ein drittes Maskenbild (13) des Zielobjekts aufgenommen wird, und
   - aus wenigstens einem nach dem dritten Maskenbild (13) mit dem veränderten Parameterwert aufgenommenen zweiten Untersuchungsbild (14, 17) des Zielobjekts durch Subtraktion des dritten Maskenbildes (13) wenigstens ein drittes Subtraktionsbild (15, 18) des Zielobjekts erzeugt wird, und
   - das wenigstens eine neue Roadmap-Bild (16, 20, 23) des Zielobjekts nach Veränderung des Parameterwertes erzeugt wird unter Verwendung des ersten Subtraktionsbildes (5) und des wenigstens einen dritten Subtraktionsbildes (15, 18).

2. Verfahren nach Anspruch 1, **dadurch gekennzeichnet, dass**

   - automatisch ein Hilfssubtraktionsbild (12) erzeugt wird, indem von einem, insbesondere zuletzt, vor dem Verändern des Parameterwertes aufgenommenen Untersuchungsbild des Zielobjekts das zweite Maskenbildes (6) oder das erste Maskenbild (3) subtrahiert wird, und
   - automatisch ein kombiniertes Subtraktionsbild (22) erzeugt wird durch Kombinieren des Hilfssubtraktionsbildes (12) mit dem dritten Subtraktionsbild (15, 18), und
   - das kombinierte Subtraktionsbild (22) zusammen mit dem ersten Subtraktionsbild (5) zum Erzeugen des neuen Roadmap-Bildes (16, 20, 23) verwendet wird.

3. Verfahren nach Anspruch 2, **dadurch gekennzeichnet, dass** durch das Gerät auf einen Empfang eines Steuerbefehls zum Verändern des Parameterwertes hin vor dessen Ausführung automatisch ein Hilfsuntersuchungsbild des Zielobjekts aufgenommen wird und dieses Hilfsuntersuchungsbild als das, insbesondere zuletzt, vor dem Verändern des Parameterwertes aufgenommene Untersuchungsbild zum Erzeugen des Hilfssubtraktionsbildes (12) verwendet wird.

**4.** Verfahren nach einem der Ansprüche 2 und 3, **dadurch gekennzeichnet, dass** das Hilfssubtraktionsbild (12) und das kombinierte Subtraktionsbild (22) als jeweils mittelwertfreie Bilder erzeugt werden, sodass ein jeweiliger Mittelwert von deren Pixelwerten Null ist.

**5.** Verfahren nach einem der Ansprüche 2 bis 4, **dadurch gekennzeichnet, dass** zum Erzeugen des kombinierten Subtraktionsbildes (22) das dritte Subtraktionsbild (15, 18) zu dem Hilfssubtraktionsbild (12) addiert wird.

**6.** Verfahren nach einem der Ansprüche 2 bis 5, **dadurch gekennzeichnet, dass** zum Erzeugen des kombinierten Subtraktionsbildes (22) vor dem Kombinieren des Hilfssubtraktionsbildes (12) mit dem dritten Subtraktionsbild (15, 18) automatisch Pixelwerte des Hilfssubtraktionsbildes (12) mit einem von 1 verschiedenen vorgegebenen Faktor multipliziert werden.

**7.** Verfahren nach einem der Ansprüche 2 bis 6, **dadurch gekennzeichnet, dass** zum Erzeugen des kombinierten Subtraktionsbildes (22) ein Farbwert des Hilfssubtraktionsbildes (12) und/oder des dritten Subtraktionsbildes (15, 18) automatisch verändert wird, sodass ein aus dem Hilfssubtraktionsbild (12) stammender Anteil (9) des kombinierten Subtraktionsbildes (22) eine andere Darstellungsfarbe aufweist als ein aus dem dritten Subtraktionsbild (15, 18) stammender Anteil (19) des kombinierten Subtraktionsbildes (22).

**8.** Verfahren nach einem der Ansprüche 2 bis 7, **dadurch gekennzeichnet, dass** zum Erzeugen des kombinierten Subtraktionsbildes (22) vor dem Kombinieren des Hilfssubtraktionsbildes (12) mit dem dritten Subtraktionsbild (15, 18) automatisch

- durch einen Vergleich des Hilfssubtraktionsbildes (12) mit dem dritten Subtraktionsbild (15, 18) ein Bildbereich (19) bestimmt wird, in welchem das dritte Subtraktionsbild (15, 18) einen in dem Hilfssubtraktionsbild (12) nicht vorhandenen Bildinhalt (19) aufweist, und
- auf einen dem bestimmten Bildbereich (19) entsprechenden Teilbereich des Hilfssubtraktionsbildes (12) ein vorgegebener Schwellenwertfilter zur Rauschunterdrückung angewendet wird.

**9.** Verfahren nach einem der Ansprüche 2 bis 8, **dadurch gekennzeichnet, dass** zum Erzeugen des Hilfssubtraktionsbildes (12) automatisch

- durch das bildgebende Gerät nach einem Empfang eines Steuerbefehls zum Verändern des Parameterwertes vor dessen Ausführung automatisch mehrere Hilfsuntersuchungsbilder des Zielobjekts aufgenommen werden, und
- diese mehreren Hilfsuntersuchungsbilder gemittelt werden, um ein Rauschen in dem Hilfssubtraktionsbild (12) zu reduzieren.

**10.** Verfahren nach Anspruch 9, **dadurch gekennzeichnet, dass** zum Aufnehmen der mehreren Hilfsuntersuchungsbilder automatisch eine Bildaufnahmefrequenz des Geräts gegenüber einem bis dahin verwendeten Wert erhöht wird.

**11.** Datenspeicher mit einem darin gespeicherten Programmcode, welcher die Verfahrensschritte eines Verfahrens gemäß einem der vorhergehenden Ansprüche kodiert und zum Durchführen des entsprechenden Verfahrens mittels einer Prozessoreinrichtung, die mit einer Datenverarbeitungseinrichtung eines bildgebenden Geräts verbunden ist, ausführbar ist.

**12.** Bildgebendes Gerät, umfassend eine Datenverarbeitungseinrichtung zum automatischen Verarbeiten von mittels des bildgebenden Geräts aufgenommenen Bildern eines Zielobjekts, wobei die Datenverarbeitungseinrichtung einen Datenspeicher nach Anspruch 11 und eine mit diesem verbundene Prozessoreinrichtung zum Ausführen des in dem Datenspeicher gespeicherten Programmcodes umfasst.

**Claims**

**1.** Method for generating at least one new roadmap image (16, 20, 23) of a target object visualised by means of an imaging device, comprising the method steps:

- generating a first roadmap image (11) by superimposition of a first (5) and a second subtraction image (9), wherein the first subtraction image (5) is generated from a first mask image (3) and contrast agent image (4) of the target object which are acquired in each case by means of the device, and the second subtraction image (9) is generated from a second mask image (6) and first examination image (7) of the target object which are acquired in each case by means of the device,

**characterised in that**
after generation of the first roadmap image (11)

- a parameter value of a physical parameter of the device which affects the acquisition of images of the target object by means of the device is changed (t5),
- immediately after the changing of the parameter value, a third mask image (13) of the target object is acquired automatically by the device using the changed parameter value, and
- from at least one second examination image (14, 17) of the target object acquired after the third mask image (13) using the changed parameter value, at least one third subtraction image (15, 18) of the target object is generated by subtraction of the third mask image (13), and
- after changing of the parameter value, the at least one new roadmap image (16, 20, 23) of the target object is generated using the first subtraction image (5) and the at least one third subtraction image (15, 18).

2. Method according to claim 1, **characterised in that**

- an auxiliary subtraction image (12) is generated automatically by subtracting the second mask image (6) or the first mask image (3) from an examination image of the target object acquired, in particular most recently, prior to the changing of the parameter value, and
- a combined subtraction image (22) is generated automatically by combining the auxiliary subtraction image (12) with the third subtraction image (15, 18), and
- the combined subtraction image (22) is used together with the first subtraction image (5) to generate the new roadmap image (16, 20, 23).

3. Method according to claim 2, **characterised in that**, in response to receiving a control command to change the parameter value, prior to the execution thereof an auxiliary examination image of the target object is acquired automatically by the device and this auxiliary examination image is used as the examination image acquired, in particular most recently, prior to the changing of the parameter value for generating the auxiliary subtraction image (12).

4. Method according to one of claims 2 and 3, **characterised in that** the auxiliary subtraction image (12) and the combined subtraction image (22) are generated as zero-mean images, so that a respective average of the pixel values thereof is zero.

5. Method according to one of claims 2 to 4, **characterised in that** the third subtraction image (15, 18) is added to the auxiliary subtraction image (12) to generate the combined subtraction image (22).

6. Method according to one of claims 2 to 5, **characterised in that**, to generate the combined subtraction image (22), pixel values of the auxiliary subtraction image (12) are automatically multiplied by a factor other than 1 prior to the combining of the auxiliary subtraction image (12) with the third subtraction image (15, 18).

7. Method according to one of claims 2 to 6, **characterised in that**, to generate the combined subtraction image (22), a colour value of the auxiliary subtraction image (12) and/or of the third subtraction image (15, 18) is automatically changed so that the portion (9) of the combined subtraction image (22) originating from the auxiliary subtraction image (12) has a display colour different from that of the portion (19) of the combined subtraction image (22) originating from the third subtraction image (15, 18).

8. Method according to one of claims 2 to 7, **characterised in that**, to generate the combined subtraction image (22), the following occur automatically prior to the combining of the auxiliary subtraction image (12) with the third subtraction image (15, 18):

- by comparison of the auxiliary subtraction image (12) with the third subtraction image (15, 18), an image region (19) is determined in which the third subtraction image (15, 18) has an image content (19) not present in the auxiliary subtraction image (12), and
- a predefined threshold value filter is applied to a subregion of the auxiliary subtraction image (12) corresponding to the particular image region (19) for noise suppression.

9. Method according to one of claims 2 to 8, **characterised in that**, to generate the auxiliary subtraction image (12), the following occur automatically:

- after receiving a control command to change the parameter value, prior to the execution thereof a plurality of auxiliary examination images of the target object are acquired automatically by the imaging device, and
- this plurality of auxiliary examination images is averaged in order to reduce noise in the auxiliary subtraction image (12).

10. Method according to claim 9, **characterised in that**, to acquire the plurality of auxiliary examination images, an image acquisition frequency of the device is automatically increased compared to a value used hitherto.

11. Data storage device having stored program code therein which encodes the procedural steps of a method according to one of the preceding claims and which can be executed to carry out the corresponding method by means of a processor device connected to a data processing device of an imaging device.

12. Imaging device, comprising a data processing device for automatically processing target object images acquired by means of the imaging device, wherein the data processing device comprises a data storage device according to claim 11 and, connected thereto, a processor device for executing the program code stored in the data storage device.

**Revendications**

1. Procédé de production d'au moins une nouvelle image (16, 20, 23) de cartographie d'un objet cible représenté au moyen d'un appareil d'imagerie, comprenant les stades de procédé

- production d'une première image (11) de cartographie par superposition d'une première (5) et d'une deuxième image (9) de soustraction, dans lequel on produit la première image (5) de soustraction à partir de respectivement une première image (3) de masque enregistrée au moyen de l'appareil et d'une image (4) d'agent de contraste de l'objet cible et on produit la deuxième image (9) de soustraction à partir respectivement d'une deuxième image (6) de masque enregistrée au moyen de l'appareil et d'une première image (7) d'examen de l'objet cible,

**caractérisé en ce qu'**
après la production de la première image (11) de cartographie

- on modifie (t5) une valeur d'un paramètre physique de l'appareil, qui influe sur l'enregistrement d'images de l'objet cible au moyen de l'appareil,
- immédiatement après la modification de la valeur du paramètre et en utilisant la valeur modifiée du paramètre, on enregistre automatiquement par l'appareil une troisième image (13) de masque de l'objet cible, et
- à partir d'au moins une deuxième image (14, 17) d'examen de l'objet cible enregistrée après la troisième image (13) de masque avec la valeur modifiée du paramètre, on produit par soustraction de la troisième image (13) de masque au moins une troisième image (15, 18) de soustraction de l'objet cible, et
- on produit la au moins une nouvelle image (16, 20, 23) de cartographie de l'objet cible après modification de la valeur du paramètre en utilisant la première image (5) de soustraction et la au moins une troisième image (15, 18) de soustraction.

2. Procédé suivant la revendication 1, **caractérisé en ce que**

- l'on produit automatiquement une image (12) de soustraction auxiliaire en soustrayant la deuxième image (6) de masque ou la première image (3) de masque d'une image d'examen de l'objet cible enregistrée, notamment en dernier, avant la modification de la valeur du paramètre, et
- l'on produit automatiquement une image (22) de soustraction combinée en combinant l'image (12) de soustraction auxiliaire à la troisième image (15, 18) de soustraction, et
- l'on utilise l'image (22) de soustraction combinée ensemble avec la première image (5) de soustraction pour la production de la nouvelle image (16, 20, 23) de cartographie.

**3.** Procédé suivant la revendication 2, **caractérisé en ce que** l'on enregistre par l'appareil, à la réception d'une instruction de commande de modification de la valeur du paramètre avant son exécution, automatiquement une image auxiliaire d'examen de l'objet cible et on utilise cette image d'examen auxiliaire comme l'image d'examen enregistrée, notamment en dernier, avant la modification de la valeur du paramètre pour la production de l'image (12) de soustraction auxiliaire.

**4.** Procédé suivant l'une des revendications 2 et 3, **caractérisé en ce que** l'on produit l'image (12) de soustraction auxiliaire et l'image (22) de soustraction combinée comme respectivement des images sans valeur moyenne, de sorte qu'une valeur moyenne respective de leurs valeurs de pixel est égale à zéro.

**5.** Procédé suivant l'une des revendications 2 à 4, **caractérisé en ce que**, pour la production de l'image (22) de soustraction combinée, on additionne la troisième image (15, 18) de soustraction à l'image (12) de soustraction auxiliaire.

**6.** Procédé suivant l'une des revendications 2 à 5, **caractérisé en ce que**, pour la production de l'image (22) de soustraction combinée, on multiplie, avant la combinaison de l'image (12) de soustraction auxiliaire à la troisième image (15, 18) de soustraction, automatiquement des valeurs de pixels de l'image (12) de soustraction auxiliaire par un facteur donné à l'avance différent de 1.

**7.** Procédé suivant l'une des revendications 2 à 6, **caractérisé en ce que**, pour la production de l'image (22) de soustraction combinée, on modifie une valeur de couleur de l'image (12) de soustraction auxiliaire et/ou de la troisième image (15, 18) de soustraction automatiquement, de manière à ce qu'une proportion (9) provenant de l'image (12) de soustraction auxiliaire, de l'image (22) de soustraction combinée ait une autre couleur de présentation qu'une proportion (19), provenant de la troisième image (15, 18) de soustraction, de l'image (22) de soustraction combinée.

**8.** Procédé suivant l'une des revendications 2 à 7, **caractérisé en ce que**, pour la production de l'image (22) de soustraction combinée, avant la combinaison de l'image (12) de soustraction auxiliaire à la troisième image (15, 18) de soustraction automatiquement

- par une comparaison de l'image (12) de soustraction auxiliaire à la troisième image (15, 18) de soustraction, on détermine un domaine (19) d'image, dans lequel la troisième image (15, 18) de soustraction a un contenu (19) d'image, qui n'est pas présent dans l'image (12) de soustraction auxiliaire, et
- on applique, pour la suppression du bruit, un filtre de valeur de seuil donné à l'avance à un domaine partiel correspondant au domaine (19) d'image déterminé de l'image (12) de soustraction auxiliaire.

**9.** Procédé suivant l'une des revendications 2 à 8, **caractérisé en ce que**, pour la production de l'image (12) de soustraction auxiliaire automatiquement

- par l'appareil d'imagerie, après une réception d'une instruction de commande de modification de la valeur du paramètre, avant son exécution, automatiquement on enregistre plusieurs images d'examen auxiliaires de l'objet cible, et
- on fait la moyenne de ces plusieurs images d'examen auxiliaires pour réduire un bruit dans l'image (12) de soustraction auxiliaire.

**10.** Procédé suivant la revendication 9, **caractérisé en ce que**, pour l'enregistrement des plusieurs images d'examen auxiliaires, on augmente automatiquement une fréquence d'enregistrement d'image de l'appareil par rapport à une valeur utilisée jusqu'ici.

**11.** Mémoire de données ayant un code de programme, qui y est mis en mémoire et qui code les stades d'un procédé suivant l'une des revendications précédentes et qui peut être réalisé pour effectuer le procédé correspondant au moyen d'un dispositif de processeur, qui est relié à un dispositif de traitement de données d'un appareil d'imagerie.

**12.** Appareil d'imagerie, comprenant un dispositif de traitement de données pour le traitement automatique d'images, enregistrées au moyen de l'appareil d'imagerie, d'un objet cible, dans lequel le dispositif de traitement de données comprend une mémoire de données suivant la revendication 11 et un dispositif processeur relié à celle-ci pour la réalisation du code de programme mis dans la mémoire de données.

FIG 1

FIG 2

FIG 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20170228863 A1